# FASCICULE DE BREVET EUROPEEN

(11) **EP 1 226 135 B1**
(45) Date de publication et mention de la délivrance du brevet: **29.12.2004**
(21) Numéro de dépôt: 00969645.1
(22) Date de dépôt: 19.10.2000
(51) Int. Cl.: C07D 333/16

(54) **PROCEDE DE PREPARATION D'UN DERIVE DU THIOPHENE**
VERFAHREN ZUR HERSTELLUNG EINES THIOPHENDERIVATES
METHOD FOR PREPARING A THIOPHENE DERIVATIVE

(30) Priorité: 22.10.1999 FR 9913205
(43) Date de publication de la demande: 31.07.2002
(73) Titulaire: Sanofi-Aventis, 75013 Paris (FR)
(72) Inventeur: BIARD, Michel, F-04200 Sisteron (FR); BOUSQUET, André, F-04200 Sisteron (FR)
(74) Mandataire: Thouret-Lemaitre, Elisabeth
(86) Numéro de dépôt international: PCT/FR2000/002909
(87) Numéro de publication internationale: WO 2001/029024

(56) Documents cités:
- WO-A-98/57974
- US-A- 4 127 580
- C G SCRETTAS: "On the Mechanism of Ring Metallation of Aromatic Compounds. Metallation of Thiophen by Lithium and by Lithium Dihydroarylides" JOURNAL OF THE CHEMICAL SOCIETY, PERKIN TRANSACTIONS 2,GB,CHEMICAL SOCIETY. LETCHWORTH, 1974, pages 745-748, XP002102778 ISSN: 0300-9580 cité dans la demande
- TRIFONOV A ET AL: "Synthesis of asymmetric beta-hydroxy-cyclopentadienyl ligands and of their bidentate lanthanide complexes" JOURNAL OF ORGANOMETALLIC CHEMISTRY,CH,ELSEVIER-SEQUOIA S.A. LAUSANNE, vol. 582, no. 2, 20 juin 1999 (1999-06-20), pages 211-217, XP004182772 ISSN: 0022-328X

## Description

La présente invention se rapporte d'une manière générale, à un procédé de préparation d'un dérivé du thiophène.

Plus particulièrement, l'invention concerne un procédé de préparation du 2-thiényl-2-éthanol de formule :

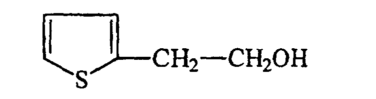

Ce composé s'est révélé intéressant comme intermédiaire de synthèse pour la préparation de divers produits chimiques, notamment des médicaments dérivés de [3,2-c]thiénopyridine utiles comme agents antiagrégants plaquettaires et anti-thrombotiques.

Parmi les dérivés de [3,2-c]thiénopyridine doués de telles propriétés, on peut citer en particulier la 5-(2-chloro-benzyl)-4,5,6,7-tétrahydro [3,2-c]thiénopyridine ou ticlopidine (DCI) ainsi que l'α-(4,5,6,7-tétrahydro [3,2-c]-thiéno-5-pyridyl) (2-chlorophényl)acétate de méthyle plus particulièrement sous la forme de son énantiomère dextrogyre ou clopidogrel.

On connaît divers procédés pour la préparation industrielle du 2-thiényl-2-éthanol. L'un des plus utilisés est sans conteste, le procédé décrit dans le brevet US 4127580 impliquant la métallation du thiophène au moyen de butyllithium, le traitement du dérivé 2-lithié ainsi obtenu avec l'oxyde d'éthylène et l'hydrolyse du composé formé de manière à produire le composé souhaité.

Toutefois, ce procédé n'est pas exempt d'inconvénients en raison d'une part de la production de butane inhérente à l'utilisation du butyllithium et d'autre part du coût relativement élevé de cet agent métallant qui, proportionnellement représente une fraction importante du prix de revient des dérivés de [3,2-c]thiénopyridine obtenus en finalité.

La préparation du 2-thiényl-2-éthanol selon un procédé industriellement rentable et susceptible d'éviter les inconvénients du procédé antérieur ci-dessus reste d'un intérêt incontestable.

Divers exemples de métallation du thiophène au départ d'un métal alcalin ont été rapportés dans la littérature chimique.

On a décrit, par exemple la préparation de l'acide 2-thiényl-carboxylique par métallation transitoire du thiophène au moyen du complexe naphtalène-sodium puis carbonatation du dérivé sodé pour obtenir l'acide en question.

Le rendement en acide ainsi formé n'a été cependant que de 40%. Une réaction tout à fait analogue a été rapportée dans J. Chem. Soc. Perkin II (1974), 745-748. Toutefois, dans le procédé décrit, le sodium a été remplacé par le lithium pour donner, après carbonatation, 41% d'acide 2-thiophène-carboxylique.

Cet article mentionne cependant une modification au procédé de lithiation décrit selon lequel le thiophène est métallé par le complexe lithium-naphtalène en présence de 1,1-diphényléthylène ou d'α-méthylstyrène pour donner, après carbonatation, l'acide 2-thiophènecarboxylique avec des rendements d'au moins 77% voire supérieure à 90%.

Or, il a maintenant été trouvé, de manière surprenante, que le 2-thiényl-2-éthanol peut être obtenu avec des rendements très importants puisque de l'ordre de 80% par sodation intermédiaire du thiophène moyennant l'utilisation de sodium et d'α-méthylstyrène à l'exclusion du naphtalène.

Ainsi, selon l'invention, on prépare le 2-thiényl-2-éthanol selon un procédé impliquant les étapes suivantes :
a) métallation du thiophène au moyen d'un métal alcalin et ce, en présence d'un agent de transfert d'électron
b) traitement du composé ainsi obtenu avec l'oxyde d'éthylène
c) hydrolyse du dérivé thiényle ainsi formé, ce qui fournit le 2-thiényl-2-éthanol souhaité.

Le métal alcalin utilisé dans le procédé de l'invention peut être le lithium, le sodium ou le potassium. Toutefois, le sodium constitue un métal alcalin particulièrement préféré.

Celui-ci est habituellement et préférentiellement utilisé sous forme d'une dispersion du métal finement divisé dans un milieu non donneur d'électron, la taille des particules de sodium ainsi divisé variant de 1 à 100 microns, plus généralement de 1 à 30 microns et de préférence de 1 à 10 microns.

De telles dispersions de sodium peuvent être obtenues en chauffant à une température supérieure à 97,5°C, un mélange de sodium dans un milieu approprié, ce qui fournit un système binaire de liquides non miscibles qui peuvent être émulsifiés de la même manière que l'eau et l'huile. Par la suite, lorsque cette émulsion est refroidie sous cette température de 97,5°C, le sodium se solidifie sous forme de minuscules sphéroïdes en suspension dans le milieu en question.

Par conséquent, on peut préparer des dispersions de sodium en chauffant un mélange de sodium métallique dans un milieu approprié et ce, au-delà du point de fusion du sodium et en émulsifiant l'ensemble par agitation très rapide. Le milieu doit présenter un point d'ébullition supérieur au point de fusion du métal à moins que la dispersion ne soit préparée sous pression supérieure à la pression atmosphérique. Dans ce cas, on peut envisager l'utilisation du thiophène à la fois comme réactif mais aussi comme milieu de dispersion du sodium.

Généralement, le milieu de dispersion en question est constitué d'un ou de plusieurs hydrocarbures liquides aromatiques ou saturés tels que par exemple le toluène, un xylène ou le n-octane.

Le toluène constitue un milieu de dispersion particulièrement avantageux. De manière notamment à éviter l'agglomération du métal et en vue d'abaisser la tension superficielle, on peut ajouter, si nécessaire un ou plusieurs agents de dispersion durant la phase d'agitation lorsque le métal se trouve à une température supérieure à sa température de fusion.

Alternativement, on peut introduire ces agents de dispersion dans le milieu de dispersion avant ou simultanément à l'ajout du métal.

Comme agents de dispersion, on utilise généralement des acides gras supérieurs comportant, de préférence, au moins 15 atomes de carbone tels que par exemple l'acide oléique, des alcools gras supérieurs ou des esters de poids moléculaire élevé, ces composés étant dotés de préférence, d'au moins 15 atomes de carbone.

On peut aussi utiliser un polymère tel que le polyéthylène, qui aura notamment pour effet d'augmenter fortement la stabilité du milieu de dispersion.

Ces agents de dispersion seront utilisés habituellement à raison, de 0,5% à 1% du poids de métal utilisé.

D'autre part, la réaction de métallation s'effectue en l'absence d'eau et sous atmosphère inerte généralement à une température pouvant varier de 0°C à + 40°C par exemple à une température de 0°C à + 30°C et de préférence à une température de l'ordre de 0°C à + 10°C.

Cette réaction se déroule avantageusement dans un solvant de préférence un éther tel que par exemple le tétrahydrofuranne ou le diméthoxyéthane et de préférence en présence d'un léger excès de thiophène tel que de 1,2 à 1,5 mole par mole de métal.

En outre, cette réaction de métallation s'effectue en présence d'un agent capable d'assurer le transfert d'un électron unique entre le métal et le thiophène. Il s'agit, en général, de composés diènes conjugués aliphatiques ou aromatiques qui peuvent être sélectionnés parmi les composés de formule: 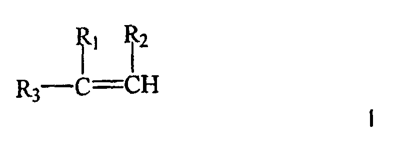 dans laquelle :
R₁ représente l'hydrogène ou un radical méthyle, éthyle ou phényle.
R₂ représente l'hydrogène ou un radical méthyle ou éthyle.
R₃ représente un radical R₄―CH=C―R₅ dans lequel R₄ et R₅, identiques
ou différents,représentent l'hydrogène ou un radical méthyle ou éthyle ou R₃ représente un groupement phényle, benzyle ou 1-phényl-1-éthyle.

Parmi les composés de formule I ci-dessus, on peut citer le 1,3-butadiène, le 2-méthyl-1,3-butadiène ou isoprène, le 1-phényléthytène ou styrène, le 1-méthyl-1-phényl-éthylëne ou α-méthyl-styrène, le 1,1-phényléthylène, le 2,3-diméthyl-1,3-butadiène, le 1,3-pentadiène et le 2,4-hexadiène.

L'isoprène, l'α-méthyl-styrène et le 2,3-diméthyl-1,3-butadiène représentent des agents de transfert d'électron unique particulièrement avantageux dans le cadre de l'invention. Toutefois, l'isoprène et plus encore l'α-méthyl-styrène représentent des diènes conjugués préférés.

Quant à la réaction avec l'oxyde d'éthylène, celle-ci se déroule de préférence, dans le milieu de métallation du thiophène et à une température variant de 0°C à la température ambiante, en particulier à une température de l'ordre de 20°C.

De même, des conditions opératoires analogues peuvent être observées pour la réaction d'hydrolyse.

Les exemples non limitatifs suivants illustrent le procédé de l'invention.

### EXEMPLE 1

### 2-Ethyl-2-éthanol

### a) Préparation de la suspension de sodium

Dans un ballon de 250ml muni d'un agitateur et d'un réfrigérant, on introduit, sous azote, 53g (61 ml) de toluène, 0,0265g de polyéthylène et 26,5g (1, 15 mole) de sodium découpé en morceaux sous azote. On chauffe à 102°C puis on agite durant 0,5 heure. On refroidit ensuite la suspension à température comprise entre 20°C et 25°C.

### b) Métallation du thiophène

Dans un réacteur de 11 à double enveloppe muni d'une ampoule d'introduction et refroidi par du dichlorométhane glacé, on charge, sous atmosphère d'azote 145,4g (133,7ml ; 1,731 mole) de thiophène. Tout en agitant on refroidit à 0°C ± 1 °C.

On dilue alors la suspension de sodium puis, on l'ajoute au thiophène. En maintenant la température à 0°C ± 1 °C, on ajoute ensuite cette suspension thiophène/sodium à un mélange de 51,8g (76ml ; 0,762 mole) d'isoprène dans 166g (187ml; 2,3 moles) de tétrahydrofuranne maintenu à 0°C ± 1°C. Dans ces conditions, la durée de l'introduction est de 1,5h environ. On maintient ensuite le milieu réactionnel à 0°C ± 1 °C pendant 2 heures.

### c) Oxyéthylènation

Au mélange obtenu au paragraphe b) ci-dessus, on ajoute, par tube plongeant, 55,8g (1,270 moles) d'oxyde d'éthylène tout en maintenant la température à 20°C ± 1°C. La durée de cette addition est de 1,5h environ. On maintient alors la suspension sous agitation durant 0,5h à 20°C ± 1°C.

### d) Hydrolyse

Dans un réacteur de 21 muni d'un système de refroidissement et d'agitation, on introduit 300ml d'eau et 74g de chlorure d'ammonium.

On refroidit cette solution à une température comprise entre -5°C et 0°C puis on transfère le milieu réactionnel obtenu au paragraphe c), par pression d'azote, en 10 à 15 minutes, sur la solution de chlorure d'ammonium.

Dans ces conditions et avec un bain réfrigérant contenant glace et méthanol la température de fin d'hydrolyse est de 20°C.

On agite pendant 15 minutes à 20°C ± 2°C, décante pendant 15 minutes et lave 3 fois à 20°C ± 2°C la phase organique (supérieure) avec 200ml d'eau. On extrait 3 fois à 20°C ± 2°C l'ensemble des phases aqueuses au moyen de 100ml (86g) de toluène.

On réunit les phases organiques et concentre la solution toluènique sous vide (50°C ; 15mmHg).

De cette manière, on obtient 133g de 2-thiényl-2-éthanol brut . Rendement : 83%.

### EXEMPLE 2

### 2-Ethyl-2-éthanol

### a) Préparation de la suspension de sodium

Dans un ballon de 250ml muni d'un agitateur et d'un réfrigérant, on introduit, sous azote, 69g (80ml) de toluène, 0,0230g d'acide oléique et 23g (1 mole) de sodium découpé en morceaux sous azote. On chauffe à 102°C puis on agite durant 0,5 heure. On refroidit ensuite la suspension à température comprise entre 20°C et 25°C.

### b) Métallation du thiophène

Dans un réacteur de 11 à double enveloppe muni d'une ampoule d'introduction et refroidi par de l'isopropanol glacé, on charge, sous atmosphère d'azote 126g (1,5 mole) de thiophène. Tout en agitant, on refroidit à 0°C ± 1 °C.

On dilue alors la suspension de sodium puis, on l'ajoute au thiophène. En maintenant la température à 0°C ± 1°C on ajoute ensuite cette suspension thiophène/sodium (76ml; 0,762 mole) à un mélange de 79g (0,670 mole) d'α-méthyl styrène dans 144g (2 moles) de tétrahydrofuranne maintenu à 0°C ± 1°C. Dans ces conditions, la durée de l'introduction est de 1,5h environ. On maintient ensuite le milieu réactionnel à 0°C ± 1 °C pendant 2 heures.

### c) Oxyéthylènation

Au mélange obtenu au paragraphe b) ci-dessus, on ajoute, par tube plongeant, 48g (1,1 mole) d'oxyde d'éthylène tout en maintenant la température à 20°C ± 1°C. La durée de cette addition est de 1,5h environ. On maintient alors la suspension sous agitation durant 0,5h à 20°C ± 1°C.

### d) Hydrolyse

Dans un réacteur de 21 muni d'un système de refroidissement et d'agitation, on introduit 230ml d'eau. On refroidit cette solution à une température comprise entre 0°C et + 5°C puis, en 10 à 15 minutes, on transfère dans l'eau le milieu réactionnel obtenu au paragraphe c). Dans ces conditions et avec un bain réfrigérant contenant glace et méthanol la température de fin d'hydrolyse est voisine de 20°C. On décante et, concentre la phase organique supérieure sous vide (50°C ; 15mmHg).

De cette manière, on obtient 239g de 2-thiényl-2-éthanol brut. Rendement : 83%.

### EXEMPLE 3

### Préparation du 2-thiényl-2-éthanol

### a) Préparation de la suspension de sodium

Dans un ballon de 250ml muni d'un agitateur et d'un réfrigérant, on introduit, sous azote, 84,3g de toluène sec, 0,32ml d'acide oléique et 28,1 g (1,2217 mole) de sodium découpé en morceaux. On chauffe à 102°C puis on agite durant 0,5heure.

On refroidit ensuite la suspension à température voisine de 25°C.

### b) Métallation du thiophène

Dans un réacteur de 11 à double enveloppe muni d'une ampoule d'introduction et refroidi, on charge, sous atmosphère d'azote, 154,2g (1,5 équivalent) de thiophène. Tout en agitant, on refroidit à 0°C et on ajoute la suspension de sodium au thiophène. En maintenant la température à 0°C ± 1°C on ajoute 67,2g (0,67 équivalent) de 2,3-diméthyl-1,3-butadiène dans 176g (2 équivalents) de tétrahydrofuranne. On maintient alors le milieu réactionnel à 0°C pendant 2 heures.

### c) Oxyéthylènation

Au mélange obtenu au paragraphe b), ci-dessus, on ajoute, par tube plongeant, 59,2g d'oxyde d'éthylène tout en maintenant la température inférieure ou égale à 20°C. On abandonne alors le milieu réactionnel durant 20 minutes à 20°C tout en assurant son agitation.

### d) Hydrolyse

Dans un réacteur de 2 litres contenant 281g de glace sous azote, on transfère, en 5 minutes et sous pression d'azote, la suspension obtenue au paragraphe c). La température du milieu en fin d'introduction est de 13°C. On agite durant 30 minutes et on décante. On extrait avec 100ml de toluène, agite durant 30 minutes et décante. On concentre alors la phase organique sous vide.

De cette manière, on obtient 145,6g de 2-thiényl-2-éthanol.
Rendement : 83%

## Revendications

1. Procédé de préparation du 2-thiényl 2-éthanol **caractérisé en ce que** :
a) l'on métalle le thiophène au moyen d'une dispersion de métal alcalin finement divisé dans un milieu non donneur d'électron et ce, en présence d'un agent de transfert d'électron
b) l'on traite le composé obtenu avec l'oxyde d'éthylène
c) l'on hydrolyse le dérivé thiényle ainsi formé
ce qui fournit le composé souhaité.

2. Procédé selon la revendication 1, **caractérisé en ce que** le métal alcalin est le sodium.

3. Procédé selon la revendication 1 ou 2, **caractérisé en ce que** le sodium divisé est sous forme de particules variant de 1 à 100 microns.

4. Procédé selon la revendication 3, **caractérisé en ce que** le sodium divisé est sous forme de particules variant de 1 à 30 microns, et de préférence de 1 à 10 microns.

5. Procédé selon l'une des revendications 1 à 4, **caractérisé en ce que** le milieu non donneur d'électron est un hydrocarbure liquide aromatique ou saturé.

6. Procédé selon l'une des revendications 1 à 5 **caractérisé en ce que** le milieu non donneur d'électron contient un ou plusieurs agents de dispersion.

7. Procédé se!on la revendication 6, **caractérisé en ce que** l'agent de dispersion est utitisé à raison de 0,6% à 1% en poids de sodium.

8. Procédé selon l'une des revendications 1 à 7, **caractérisé en ce que** la métallation s'effectue en présence d'un diène conjugué de formule générale: 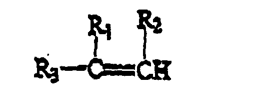 dans laquelle:
R₁ représente l'hydrogène ou un radical méthyle, éthyle ou phényle.
R₂ représente l'hydrogène ou un radical méthyle ou éthyle
R₃ représente un radical 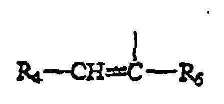 dans lequel R₄ et R₅, identiques ou différents, représentent l'hydrogène ou un radical méthyle ou éthyle ou R₃ représente un groupement phényle, benzyle ou 1-phényl-1-éthyle.

9. Procédé selon la revendication 8, **caractérisé en ce que** le diène conjugué est le 1,3-butadiène, le 2-méthyl-1,3-butadiène, le 1-phényl-éthylène, le 1-méthyl-1-phényl-éthylène, le 1,1-phényl-éthylène, le 1,3-pentadiène ou le 2,4-hexadiène.

10. Procédé selon la revendication 8, **caractérisé en ce que** le diène conjugé est le 2-méthyl-1,3-butadiène ou le 1-méthyl-1-phényl-éthylène.

11. Procédé selon une des revendications 1 à 10, **caractérisé en ce que** la métallation s'effectue à une température de 0°C à 40°C.

12. Procédé selon une des revendications 1 à 11 **caractérisé en ce que** le traitement avec l'oxyde d'éthylène et l'hydrolyse s'effectuent à une température de 0°C à la température ambiante.

## Patentansprüche

1. Verfahren zur Herstellung von 2-Thienyl-2-ethanol, **dadurch gekennzeichnet, daß** man:
a) Thiophen mit einer Dispersion von feinverteiltem Alkalimetall in einem Medium, welches keinen Elektronendonor darstellt, in Gegenwart eines Elektronentransfermittels metallisiert,
b) die erhaltene Verbindung mit Ethylenoxid behandelt und
c) das in dieser Weise gebildete Thienylderivat hydrolysiert
zur Bildung der gewünschten Verbindung.

2. Verfahren nach Anspruch 1, **dadurch gekennzeichnet, daß** das Alkalimetall Natrium ist.

3. Verfahren nach Anspruch 1 oder 2, **dadurch gekennzeichnet, daß** das verteilte Natrium in Form von Teilchen mit einer Größe von 1 bis 100 µm vorliegt.

4. Verfahren nach Anspruch 3, **dadurch gekennzeichnet, daß** das verteilte Natrium in Form von Teilchen mit einer Größe von 1 bis 30 µm, vorzugsweise 1 bis 10 µm vorliegt.

5. Verfahren nach einem der Ansprüche 1 bis 4, **dadurch gekennzeichnet, daß** das keinen Elektronendonor darstellende Medium ein aromatischer oder gesättigter flüssiger Kohlenwasserstoff ist.

6. Verfahren nach einem der Ansprüche 1 bis 5, **dadurch gekennzeichnet, daß** das keinen Elektronendonor darstellende Medium ein oder mehrere Dispergiermittel enthält.

7. Verfahren nach Anspruch 6, **dadurch gekennzeichnet, daß** das Dispergiermittel in einer Menge von 0,5 % bis 1 %, bezogen auf das Gewicht des Natriums, verwendet wird.

8. Verfahren nach einem der Ansprüche 1 bis 7, **dadurch gekennzeichnet, daß** die Metallierung in Gegenwart eines konjugierten Diens der allgemeinen Formel erfolgt: 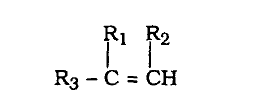 in der:
R₁ Wasserstoff oder eine Methyl-, Ethyl- oder Phenylgruppe bedeutet.
R₂ Wasserstoff oder eine Methyl- oder Ethylgruppe bedeutet,
R₃ eine Gruppe 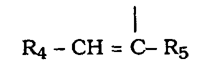 darstellt, in der R₄ und R₅, die gleichartig oder verschieden sind, Wasserstoff oder eine Methyl- oder Ethylgruppe bedeuten, oder R₃ eine Phenyl-, Benzyl- oder 1-Phenyl-1-ethylgruppe darstellt.

9. Verfahren nach Anspruch 8, **dadurch gekennzeichnet, daß** das konjugierte Dien 1,3-Butadien, 2-Methyl-1,3-butadien, 1-Phenyl-ethylen, 1-Methyl-1-phenylethylen, 1,1-Phenyl-ethylen, 1,3-Pentadien oder 2,4-Hexadien ist.

10. Verfahren nach Anspruch 8, **dadurch gekennzeichnet, daß** das konjugierte Dien 2-Methyl-1,3-butadien oder 1-Methyl-1-phenyl-ethylen ist.

11. Verfahren nach einem der Ansprüche 1 bis 10, **dadurch gekennzeichnet, daß** die Metallierung bei einer Temperatur von 0°C bis 40°C durchgeführt wird.

12. Verfahren nach einem der Ansprüche 1 bis 11, **dadurch gekennzeichnet, daß** die Behandlung mit Ethylenoxid und die Hydrolyse bei einer Temperatur von 0°C bis Umgebungstemperatur durchgeführt werden.

## Claims

1. Process for preparing 2-thienyl-2-ethanol, **characterized in that**:
a) thiophene is metallated using a dispersion of a finely divided alkali metal in a medium that is not electron-donating, in the presence of an electron transfer agent,
b) the compound obtained is treated with ethylene oxide,
c) the thienyl derivative thus formed is hydrolysed, to give the desired compound.

2. Process according to Claim 1, **characterized in that** the alkali metal is sodium.

3. Process according to Claim 1 or 2, **characterized in that** the divided sodium is in the form of particles ranging from 1 to 100 microns.

4. Process according to Claim 3, **characterized in that** the divided sodium is in the form of particles ranging from 1 to 30 microns and preferably from 1 to 10 microns.

5. Process according to one of Claims 1 to 4, **characterized in that** the medium that is not electron-donating is a liquid aromatic or saturated hydrocarbon.

6. Process according to one of Claims 1 to 5, **characterized in that** the medium that is not electron-donating contains one or more dispersants.

7. Process according to Claim 6, **characterized in that** the dispersant is used in a proportion of from 0.5% to 1% by weight of sodium.

8. Process according to one of Claims 1 to 7, **characterized in that** the metallation is carried out in the presence of a conjugated diene of general formula: 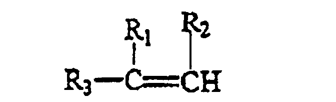 in which:
R₁ represents hydrogen or a methyl, ethyl or phenyl radical,
R₂ represents hydrogen or a methyl or ethyl radical,
R₃ represents a radical 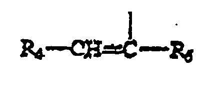 in which R₄ and R₅, which may be identical or different, represent hydrogen or a methyl or ethyl radical or R₃ represents a phenyl, benzyl or 1-phenyl-1-ethyl group.

9. Process according to Claim 8, **characterized in that** the conjugated diene is 1,3-butadiene, 2-methyl-1,3-butadiene, 1-phenylethylene, 1-methyl-1-phenylethylene, 1,1-phenylethylene, 1,3-pentadiene or 2,4-hexadiene.

10. Process according to Claim 8, **characterized in that** the conjugated diene is 2-methyl-1,3-butadiene or 1-methyl-1-phenylethylene.

11. Process according to one of claims 1 to 10, **characterized in that** the metallation is carried out at a temperature of from 0°C to 40°C.

12. Process according to one of Claims 1 to 11, **characterized in that** the treatment with ethylene oxide and the hydrolysis are carried out at a temperature of from 0°C to room temperature.
